# EUROPEAN PATENT APPLICATION

(11) **EP 2 689 724 A1**
(43) Date of publication of application: **29.01.2014**
(21) Application number: 12765883.9
(22) Date of filing: 26.03.2012
(51) Int. Cl.: A61B 5/22, A61B 1/303

(54) **DEVICE FOR MEASURING THE MUSCULAR FORCE OF THE PELVIC FLOOR**

(30) Priority: 25.03.2011 ES 201130449
(71) Applicant: Universitat Politècnica De Catalunya, 08034 Barcelona (ES); Althaia, Xarxa Assistencial De Manresa, Fundació Privada, 08243 Manresa (Barcelona) (ES)
(72) Inventor: PEÑA PITARCH, Esteban, E-08034 Barcelona (ES); ROMERO CULLERÉS, Georgia, E-08034 Barcelona (ES)
(74) Representative: Carvajal y Urquijo, Isabel
(86) International application number: PCT/ES2012/070201
(87) International publication number: WO 2012/131136

(57) **Abstract**

The invention relates to a device for measuring the muscular strength of the pelvic floor, comprising a speculum (1) consisting of two pivotally coupled parts (11, 12), each part having a grip area (31, 32) and a front area (41, 42) that is to be introduced into the vagina. A surface electrode movement sensor (2) is coupled to the grip area (32) of the speculum, with a spring (21) having a known constant K, said spring (21) having a wire diameter of between 0.5 and 1 mm, said sensor being associated with a module for reading (50) the movement. The device according to the invention also has a system for measuring parasite muscular activity by means of surface electrodes (52) associated with a strength reading module (51).

## Description

### Object of the invention

The proposed invention relates to a device for measuring the muscular strength of the pelvic floor, comprising a speculum to which is coupled a movement sensor, capable of quantifying said strength in an initial examination. Surface electrodes are also added in order to be able to eliminate the parasite muscle activity during contraction of the pelvic floor, such as abdominals, abductors and glutei. In this way, the results can be assessed and evaluated in an objective, accurate and analytical manner.

### Background of the invention

Urinary incontinence is a significant medical and social problem with an increasing trend owing to, amongst other reasons, ageing of the population where it is estimated that around two hundred million adults suffer from incontinence.

Urinary incontinence is largely prevalent in the adult population and two to four times more common in women than in men. It increases almost linearly with age until it is considered as one of the geriatric syndromes, both due to its increased prevalence in those older than 65 and to the negative impact it has on the elder person suffering from it. It significantly deteriorates the quality of life of the patients, limits their independence and reduces their self-confidence.

The current method of evaluating the pelvic floor is based on an entirely empirical system. The muscular strength of the pelvic floor, a variable directly related to urinary incontinence, the current forms of measurement are based on manual digital touch, a subjective method and measurements of pressure by means of manometers.

Manual vaginal palpation, in order to quantify it, is based on the modified Oxford scale, having values of 0 to 5, where 0 indicates no muscular contraction and 5 maximum muscular strength. There is no systematic review for being able to determine which vaginal palpation is the best method (mono, bidigital, etc.). For these reasons, it must only be used to quantitatively determine whether or not there exists muscular contraction and whether or not this is correct.

Manometry must always be measured precisely in the same anatomical area. There are currently a plurality of vaginal devices for measuring pressures, all of different sizes and technical parameters without being validated and having different pressure scales, mm of mercury or centimeters of water. This prevents results obtained using different method from being comparable.

Speculum dynamometers can measure the muscular strength of the pelvic floor directly and could be the more appropriate method for measuring said strength than the manometers current pressure transducers.

However, these dynamometers have disadvantages as the values may also become affected by the increases in intra-abdominal pressure or contractions of other muscles. There must exist a mechanism capable of controlling the synergies of the abdominals, glutei and adductors, etc.

Hitherto, none of the existing dynamometer models allow the actual muscular activity of the pelvic floor to be controlled.

### Description of the invention

The proposed invention solves the problem outlined above in an entirely satisfactory manner. The invention is a device for measuring the muscular strength of the pelvic floor, being capable of quantifying said strength in an initial examination of a woman who goes to the consultation having incontinence. In this way, the objective results can be assessed/evaluated after applying a therapeutic technique using rehabilitation and carrying out scientific research studies on urinary incontinence.

The system comprises two parts, the speculum itself, consisting of two pivoting parts, and a movement sensor coupled with a spring having a known rigidity constant K. The reading obtained, that is to say, the distance the spring is compressed is the movement.

The spring may have a wire diameter of between 0.5 and 1 mm, different values of the rigidity constant K are thereby obtained with the same wire material. Initial values for basal tone, as a function of each patient, are used as the starting point.

In order to discriminate the parasite muscles, abdominals, adductors and glutei, the device may also provide a reading by means of surface electrodes which record the muscular activity of said muscles.

### Description of the drawings

In order to complete the description and with the object of providing a greater understanding of the characteristics of the invention, according to a preferred exemplary embodiment thereof, a set of drawings are provided as an integral part of this description, wherein the following is depicted in an illustrative and non-limiting manner:

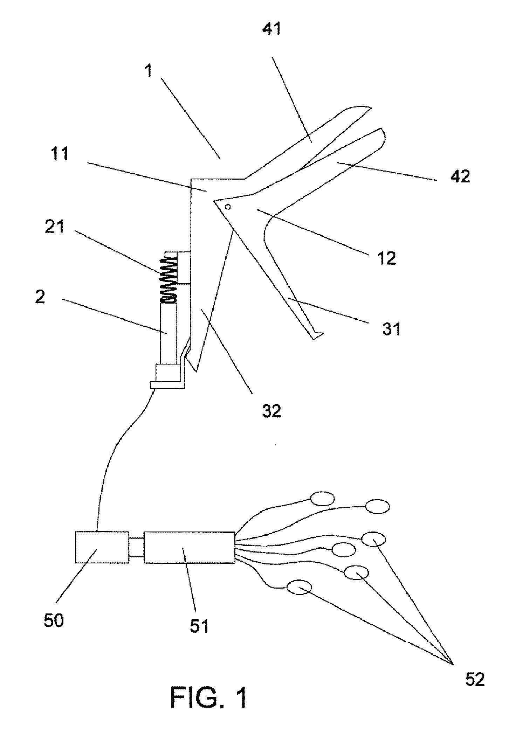

Figure 1.- shows an elevated view of the device according to the invention.
Figure 2.- shows the dispersion graphics of the two consecutive measurements

### Preferred embodiment of the invention

The invention described below is a device for measuring the muscular strength of the pelvic floor. This device allows objective data of the musculature of the pelvic floor to be obtained, thereby enabling the results to be evaluated and assessed in a credible manner.

The device for measuring the muscular strength of the pelvic floor described here comprises a speculum (1), consisting of two pivotally coupled parts (11, 12), each part having a grip area (31, 32) and a front area (41, 42) that is to be introduced into the vagina.

A movement sensor (2) is coupled to this front area (41, 42) of the speculum, introduced into the vagina, with a spring (21) having a known constant K, obtaining the value of said movement in a module for reading the movement (50). The reading obtained is therefore the movement, that is to say, the extent to which the spring (21) is compressed. This spring (21) may have a diameter of between 0.5 and 1 mm, different values of the rigidity constant K are thereby obtained with the same wire material. Initial values for basal tone, as a function of each patient, are used as the starting point.

The device may also be provided with a system for measuring the parasite strengths by means of surface electrodes (52). These surface electrodes (52) record the muscular activity of the parasite muscles, abdominals, adductors and glutei by means of a module for reading strengths (51). In this way, those values obtained are discriminated, obtaining a more objective, accurate and analytical reading.

The measuring system comprises a small handheld battery with a plurality of channels where surface electrodes (52) are arranged, as well as the module for reading strengths (51). It may be used to measure the parasite strengths, to stimulate said muscles or even for both purposes and the rehabilitation of said muscles.

### Practical exemplary embodiment:

In a sampling of 39 women with different degrees and types of urinary incontinence, the following measurements were carried out:
- Bidigital vaginal palpation quantified by the modified Oxford scale.
- Two consecutive measurements of the contraction strength of the pelvic floor registered by the device according to the invention.

The complete operation of introducing the speculum or the device according to the invention and registering the strength carried out on the same is performed for each measurement. The strength of the contraction is the result of the difference between the strength during the execution of the contraction order of the pelvic floor and the strength registered when at rest. The measurement units used are microvolts. Using the appropriate conversion, the values may be given in Newton (N).

One single observer has carried out all of the measurements. In this preliminary examination, only the data referring to the part of the system including the speculum according to the invention are registered. The effect of the parasite muscles is not included in the measurement.

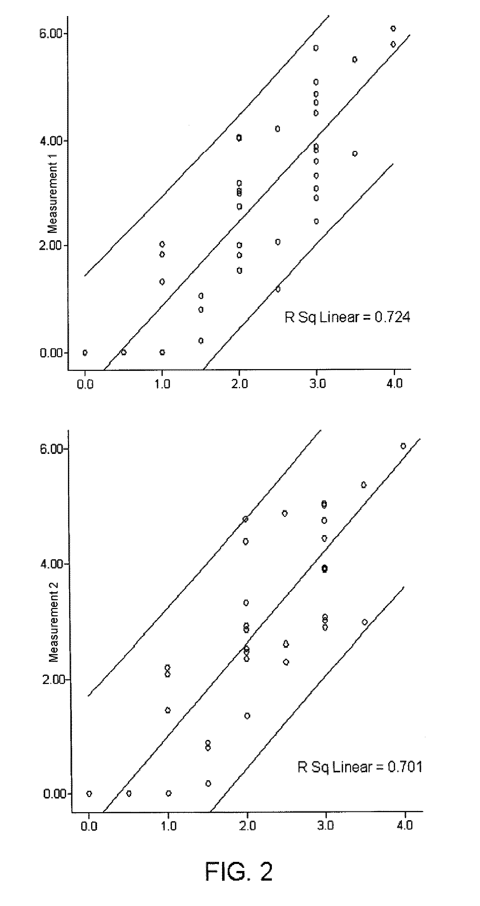

Figure 2 shows the dispersion graphics of the two consecutive measurements alongside the straight lines of regression. Confidence intervals are added to 95%.

### Pearson's correlation coefficient (r)

Measurement 1 r=0.851; Sig. (bilateral) p<0.001
Measurement 2 r=0.837; Sig. (bilateral) p<0.001

Carrying out the test with related samples indicates that a high correlation exists between the individual strengths values registered in both measurements.

**Table 1**

| | | Average | N | Typ. Deviation | Typ. error in the average |
|---|---|---|---|---|---|
| Measurements | 1 | 2.9582 | 39 | 1.83559 | .29393 |
| | 2 | 3.1262 | 39 | 1.90179 | .30453 |

**Table 2**

| | | N | Correlation | Sig. |
|---|---|---|---|---|
| Measurements | 1 and 2 | 39 | .958 | .000 |

These data provide preliminary evidence relating to the strong positive correlation existing between the modified Oxford scale and the contraction strength values of the pelvic floor registered by the device according to the invention.

At the same time, the preliminary precision data of the measurement instrument seem relevant. There are few differences obtained in the values of the two measurements registered.

## Claims

1. Device for measuring the muscular strength of the pelvic floor, comprising a speculum (1) consisting of two pivotally coupled parts (11, 12), each part having a grip area (31, 32) and a front area (41, 42) that is to be introduced into the vagina, **characterized in that** a movement sensor (2) is coupled to the grip area (32) of the speculum with a spring (21) having a known constant K, associated with a module for reading (50) the movement.

2. Device for measuring the muscular strength of the pelvic floor according to Claim 1, **characterized in that** the spring (21) has a wire diameter of between 0.5 and 1 mm.

3. Device for measuring the muscular strength of the pelvic floor according to the preceding claims, **characterized in that** it comprises a system for measuring the parasite muscular activity by means of surface electrodes (52) associated with a strength (51) reading module.
